# EUROPEAN PATENT APPLICATION

(11) **EP 1 232 857 A2**
(43) Date of publication of application: **21.08.2002**
(21) Application number: 02003710.7
(22) Date of filing: 19.02.2002
(51) Int. Cl.: B32B 27/40, B32B 33/00, A61F 13/15

(54) **Laminate material with microporous membrane**

(30) Priority: 20.02.2001 SE 0100577; 20.02.2001 US 269385 P
(71) Applicant: SCA Hygiene Products AB, 405 03 Göteborg (SE); TYTEX A/S, 7430 Ikast (DK)
(72) Inventor: Carlbark, Olle, 41757 Göteborg (SE); Rönnberg, Peter, 43133 Mölndal (SE); Strannemalm, Kenneth, 44831 Floda (SE); Balslev Sörensen, Bettina, 8600 Silkeborg (DK)
(74) Representative: Hammond, Andrew David

(57) **Abstract**

A laminate material (25) for a garment (10) for use in absorbing bodily wastes. The laminate material has a first layer (26), a second layer (28), and a third layer (30) intermediate the first and second layer. The first layer (26) and the second layer (28) are predominantly a polyester material having a basis weight of from 70 g/m² to 150 g/m², preferably from 100 g/m² to 130 g/m², and the third layer (30) is a microporous membrane, preferably of polyurethane.

## Description

### TECHNICAL FIELD:

The present invention relates to a laminate material for a garment for use in absorbing bodily wastes according to the preamble of claim 1, and to a garment utilizing such a laminate material.

### BACKGROUND OF THE INVENTION:

Garments for use in absorbing bodily wastes may be worn by young children, as well as incontinent persons. These garments are fastened about the waist of the wearer. Such garments may be generally divided into two types; namely, a first type consisting of disposable garments and a second type consisting of washable reusable garments. Garments of the first type are generally termed disposable diapers and these usually comprise an absorbent batt sandwiched between a liquid pervious topsheet and a liquid impervious backsheet. A disposable diaper is intended to be discarded after an insult has been detected or once its absorbent capacity has been reached. A washable reusable garment is generally intended to be used with a disposable absorbent insert. Such a garment is normally termed a diaper cover and the disposable insert is held in place against the crotch region of the body of the wearer by the diaper cover. The disposable insert is discarded after an insult while the diaper cover may be reused and laundered when necessary.

Diaper covers are generally worn by incontinent adults. It is desirable that covers be discrete and comfortable when worn, cheap to manufacture and durable. They should be capable of repelling liquid to thereby reduce risk of leakage, at the same time that they are "breathable", i.e. vapour-permeable. These properties must be maintained even after repeated laundering. Since diaper covers are intended to be laundered, the covers must not shrink or lose their shape unduly.

Accordingly, there exists a need for a laminate material which is suitable for use as a diaper cover which exhibits the properties mentioned above.

### SUMMARY OF THE INVENTION:

This need is fulfilled in accordance with the present invention by a laminate material as claimed in claim 1 and by a garment according to claim 13.

Advantageous embodiments of the laminate material according to the present invention and of the garment are detailed in the respective dependent claims.

### BRIEF DESCRIPTION OF THE DRAWINGS:

The invention will be described in greater detail in the following by way of example only and with reference to embodiments shown in the attached drawings, in which:
- Fig. 1: is a schematic plan view of a first embodiment of a garment for use in absorbing bodily wastes utilizing a laminate material according to the present invention;
- Fig. 2: is a schematic sectional view along line II-II of Fig. 1 on a considerably larger scale, and
- Fig. 3: is a schematic plan view of alternative designs for the first fastening system on the garment illustrated in Fig. 1.

### DETAILED DESCRIPTION OF PREFERRED EMBODIMENTS:

In the drawings, a garment in the form of a diaper cover utilizing a laminate material of the present invention is generally denoted by reference numeral 10. The garment is delimited by a peripheral edge 12 and a longitudinal axis L divides the garment into a first lateral half 14 and a second lateral half 16. A transverse axis T perpendicular to longitudinal axis further divides the garment into a first end 18 and a second end 20. The garment 10 has an inwardly facing surface 22 which faces the wearer during use, and an outwardly facing surface 24 which faces away from the wearer during use.

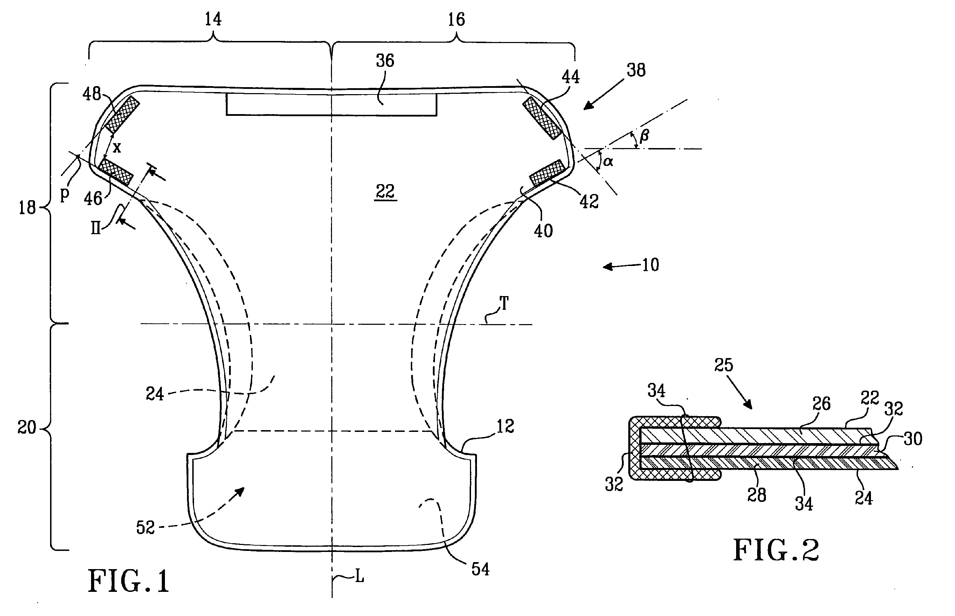

As is most clearly apparent from Fig. 2, the inwardly facing surface 22 and the outwardly facing surface 24 are associated with components of a laminate material, generally denoted by reference numeral 25. Thus, the laminate material 25 comprises a first layer 26, a second layer 28 and a third layer 30 intermediate the first and second layers, with the first layer 26 presenting the inwardly facing surface 22 and the second layer presenting the outwardly facing surface 24. For the sake of clarity, the various layers of the laminate material have been shown in Fig. 2 as having about the same thickness. It is, however, to be noted that Fig. 2 is highly schematic and, in reality, the third layer 30 has a thickness which is considerably less than that of the first and second layers.

In accordance with the present invention, the first layer 26 and the second layer 28 comprise predominantly a polyester material having a basis weight of from 70 g/m² to 150 g/m², preferably from 100 g/m² to 130 g/m², most preferably about 120 g/m², and the third layer 30 comprises a microporous membrane, preferably of polyurethane. In this respect, the expression "predominantly a polyester material" means that more than 50% of the material of these layers is polyester. It is conceivable that polyamide could be used, at least as a certain proportion. Furthermore, natural fibres such as cotton could be included in low amounts. Nevertheless, due to the suitability of polyester as a facing material, both in terms of cost and material properties (soft to touch, durable, form-retentive, etc.), in a preferred embodiment the first layer 26 and the second layer 28 comprise at least 90% polyester material, preferably essentially 100% polyester material. The expression "essentially 100% polyester material" means that the material consists essentially of 100% polyester, though may include small amounts of foreign matter. For ease of production, and irrespective of the actual material used, it is advantageous if the first layer 26 and the second layer 28 are of identical material, both in terms of dimensions and constituent materials.

The presence of a microporous membrane 30 provides the laminate material of the present invention with water-repelling properties, at the same time that the laminate material is "breathable". A suitable membrane has a basis weight of from 15 g/m² to 40 g/m², preferably from 25 g/m² to 35 g/m². In practice, a polyurethane membrane having a basis weight of about 30 g/m² has been shown to be highly suitable. The breathability, i.e. the water vapour permeability, of the laminate material is established in accordance with ASTM method E398. For optimal wearer comfort, the laminate material should exhibit a water vapour permeability value of at least 2500 g/m²/24h, preferably at least 3000 g/m²/24h, more preferably at least 3500 g/m²/24h and most preferably at least 4000 g/m²/24h.

In order not to unduly negatively affect the water vapour permeability of the third layer 30, it is necessary that lamination of the three layers 26, 28, 30 is effected such that a sufficient number of the micropores of the polyurethane membrane remain "open". Preferably, the third layer 30 is adhesively bonded to the first layer 26 and the second layer 28. More specifically, 11 g/m² to 16 g/m² of adhesive, preferably from 12.5 g/m² to 14.5 g/m² of adhesive may be used to bond both the third layer 30 to the first layer 26 over a first bonding surface 32 and the third layer to the second layer 28 over a second bonding surface 34, respectively. The adhesive may be applied in a dot-bonding pattern on each of the first and second bonding surfaces such that from 10% to 30%, preferably, 15% to 25%, most preferably 17% to 21%, of the surfaces are covered by the adhesive. A coverage of about 18 % to 19% has been shown to be highly suitable. The dot-bonding pattern may comprise of from 110 to 150 dot-bonds per square centimetre, preferably from 125 to 135 dot-bonds per square centimetre, with about 128 dot-bonds per square centimetre being advantageous. A suitable adhesive has been shown to be a polyurethane hot-melt adhesive which crosslinks through air moisture and gives a flexible film after curing. The adhesive generally exhibits a Brookfield viscosity of about 5700 mPa.s at 100 °C, about 4100 mPa.s at 110 °C and about 3100 mPa.s at 120 °C.

The above-described laminate material is eminently suitable for use in a garment such as a diaper cover. As is most clearly shown in Fig. 2, for increased comfort and durability, the peripheral edge 12 of the diaper cover 10 may be provided with braiding 32. The braiding 32 may be in the form of a strip of material of 15 - 20 mm width which is folded around the edges of the laminate material 25. Preferably, the braiding is a cotton/polyester material containing elastic threads to impart elasticity to the braiding. The braiding 32 may be stitched to the layered structure in a conventional manner using one or more lines of stitching 34.

To improve fit and comfort, the diaper cover may be provided with an elasticated waist band 36. The waist band 36 may be positioned adjacent and along the transverse edge on the first layer 26 in the first end 18 of the diaper cover. The waist band is advantageously of the same material as the braiding 32, though of somewhat greater width, for example 22 mm. The waist band is attached to the laminate material 25 in a stretched condition preferably by stitching, utilizing the line of stitching 34 which secures the braiding to the layered structure as well as a further line or lines of stitching running substantially parallel to the transverse edge.

So that the garment may be secured around the waist of a wearer, the garment is provided with a fastening system. The fastening system may be either mechanical, such as buttons and button holes, press studs and poppers or hook-and-loop systems, or adhesive. As will be described in greater detail below, in a preferred embodiment of the present invention the fastening system comprises a hook-and loop arrangement. Irrespective of the actual type of fastening system, it comprises a first fastening arrangement, generally denoted by reference numeral 38, located on the first end 18 of the garment. More particularly, the first fastening arrangement 38 is located at an outer margin 40 of at least one of the first and the second lateral halves 14, 16. The outer margin 40 may be defined as that region of the garment which extends inwardly up to 5 cm from the peripheral edge 12 of the garment. The first fastening arrangement is to be regarded as being located at this outer margin if at least a portion of the first fastening arrangement is disposed within that region. As illustrated in Fig. 1, the first fastening arrangement 38 preferably comprises two outwardly facing edges 42, 44 forming an angle α therebetween. The expression "outwardly facing edges" implies those edges which generally follow a portion of the peripheral edge 12 of the garment. Thus, a first one edge 42 of the outwardly facing edges faces towards the transverse axis T, while a second one edge 44 of the outwardly facing edges faces away from the transverse axis T. Advantageously, to avoid an excessive amount of unsecured material along the outer margin 40, the two outwardly facing edges 42, 44 of the first fastening arrangement should lie within 2.0 cm, preferably within 1.5 cm, of the peripheral edge 12 of the garment.

In a preferred embodiment of the present invention, the angle α between the two outwardly facing edges 42, 44 is from 45 ° to 85 °, preferably from 50 ° to 80 °, more preferably from 55 ° to 75 ° and most preferably from 60 ° to 70 °. By angling the outwardly facing edges 42, 44 in this manner, a more compact arrangement is attainable. Furthermore, by having the outwardly facing edges 42, 44 run substantially parallel to the peripheral edge 12 of the garment in the outer margin 40, the garment will taper towards a point in this region, thereby making it easier for a user to grasp the entire outer margin in one hand.

In a further preferred embodiment of the invention, the first one edge 42 of the two outwardly facing edges subtends an angle β to the transverse axis T (or at least to a line parallel to the transverse axis T as illustrated) of from 0 ° to 50 °, preferably from 15 ° to 40 ° and more preferably from 25 ° to 35 °. Without being bound to any theory, it is believed that by angling the first one edge 42 in this manner, at least a part of the first fastening arrangement may be aligned with those tensions which arise along the peripheral edge of the garment in the leg region, thereby being better able to accommodate these forces.

In Fig. 1, the first fastening arrangement 38 is shown as having two distinct patches 46, 48 of fastening material. It is to be understood, of course, that a corresponding effect can be achieved using a single patch having two outwardly facing edges angled in the manner described above. In the case where two or more patches of fastening material are utilized, the patches 46, 48 should be separated by a distance x of not more than 6.0 cm, preferably not more than 3.5 cm and most preferably not more than 2.0 cm. In other words, the closest distance between proximal points on adjacent patches should be no greater than the above recited distances.

Due to the provision of the angle α, virtual extensions of the two outwardly facing edges 42, 44 will intersect at a point P lying externally of the peripheral edge 12. Preferably, the point P is located at a distance of from 1.0 cm to 10.0 cm, more preferably from 2.5 cm to 5.0 cm, from the peripheral edge 12.

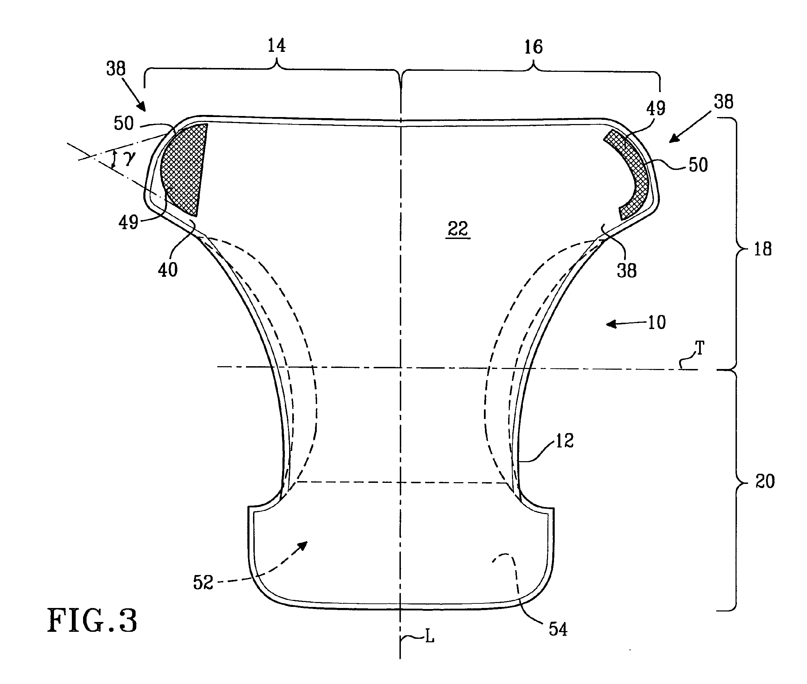

The advantages associated with the above-described embodiment may also be enjoyed by embodiments in which the first fastening arrangement presents a curved outwardly facing edge as opposed to two angled edges. Thus, Fig. 3 illustrates alternative embodiments of the present invention in which, for the sake of brevity, a garment is shown having a first shape of first fastening arrangement in the first lateral half 14 and a second shape of first fastening arrangement in the second lateral half 16. In both cases, the first fastening arrangement is illustrated as a single patch 49 of fastening material having an outwardly facing edge 50. The first shape is a segment of a circle while the second shape is of substantially constant width. In accordance with the invention, and irrespective of the actual shape, the outwardly facing edge 50 is curved such that there exists two points on the outwardly facing edge, tangents to which subtend an angle γ of not more than 90 °, preferably not more than 60 ° and most preferably not more than 30 °.

Advantageously, the first fastening arrangement of the illustrated embodiments comprises hook fastening material. The patches 46, 48, 49 of hook material may comprise a base tape and monofilament hooks in 100% polyester having a weight of at least 320 g/m². Since the first fastening arrangement 38 preferably comprises a hook material, the fastening system must also comprise a second fastening arrangement, generally denoted by reference numeral 52, complementary to the first fastening arrangement. In the cases in which the garment is fastened around the waist of a wearer by fastening the first end 18 of the garment directly to the second end 20, the second fastening arrangement 52 is disposed on the second end 20 of the garment. Thus, the second fastening arrangement comprises at least one panel 54 of loop material. Such material may be 100% polyamide having a basis weight of about 210 g/m². Preferably, and as illustrated in the drawings, the panel 54 extends over substantially the entire width of the second end 20 of the garment 10. Alternatively, the second fastening arrangement 52 may consist of a plurality of panels of loop material suitably distributed over the second end 20 of the garment to thereby allow adjustment of the garment to different sizes of wearer.

For ease of fastening, the second end 20 of the garment may be adapted to cover a portion of the abdomen of a wearer. In other words, the first fastening arrangement 38 is disposed on the inwardly facing surface 22 of the garment on the end 18 which will cover the buttocks of the wearer during use and the panel 54 of loop material of the second fastening arrangement 52 is disposed on the outwardly facing surface 24 of the garment on the end 20 which covers a portion of the wearer's abdomen during use. Thus, to fasten the garment, the garment is drawn between the legs of the intended wearer, the second end 20 is held against the abdomen of the wearer and the regions of the first end 18 on which the first fastening arrangement 38 is disposed are drawn around the waist of the wearer and the first fastening arrangement 38 is made to engage the second fastening arrangement. Naturally, the fastening routine can be performed with the wearer either standing or lying on his/her back.

A diaper cover comprising a laminate material of the present invention has been shown to offer high user comfort and adequate shape retention (no more than 6% shrinkage) even after being subjected to 100 wash cycles at 60 °C. In terms of shape retention, it is advantageous if the first layer 26 and the second layer 28 have an amount of shrinkage which is no more than the amount of shrinkage of the third layer 30.

It is to be understood that the invention has been described above and illustrated in the drawings purely by way of example. Accordingly, the skilled person will appreciate that the present invention may be varied within the scope of the appended claims. For example, the relative positions of the patches of hook and loop material may be interchanged.

## Claims

1. A laminate material (25) for a garment (10) for use in absorbing bodily wastes, said laminate material comprising:
a first layer (26);
a second layer (28), and
a third layer (30) intermediate said first and second layer,
**characterized in that** said first layer (26) and said second layer (28) comprise predominantly a polyester material having a basis weight of from 70 g/m² to 150 g/m², preferably from 100 g/m² to 130 g/m², and said third layer (30) comprises a microporous membrane.

2. The laminate material (25) as claimed in claim 1, **characterized in that** said first layer (26) and said second layer (28) are of identical material.

3. The laminate material (25) as claimed in claim 1 or 2, **characterized in that** said third layer (30) is a microporous polyurethane membrane.

4. The laminate material (25) as claimed in any one of claims 1 to 3, **characterized in that** said third layer (30) has a basis weight of from 15 g/m² to 40 g/m², preferably from 25 g/m² to 35 g/m².

5. The laminate material (25) as claimed in any one of the preceding claims, **characterized in that** said first layer (26) and said second layer (28) comprise at least 90% polyester material, preferably essentially 100% polyester material.

6. The laminate material (25) as claimed in any one of the preceding claims, **characterized in that** said third layer (30) is adhesively bonded to said first layer (26) and said second layer (28).

7. The laminate material (25) as claimed in claim 6, **characterized in that** from 11 g/m² to 16 g/m² of adhesive, preferably from 12.5 g/m² to 14.5 g/m² of adhesive is used to bond both said third layer (30) to said first layer (26) over a first bonding surface (32) and said third layer to said second layer (28) over a second bonding surface (34), respectively.

8. The laminate material (25) as claimed in claim 6 or claim 7, **characterized in that** said adhesive is applied in a dot-bonding pattern on each of said first and second bonding surfaces (32; 34) such that from 10% to 30%, preferably, 15% to 25%, most preferably 17% to 21%, of said surfaces are covered by said adhesive.

9. The laminate material (25) as claimed in claim 8, **characterized in that** said dot-bonding pattern comprises of from 110 to 150 dot-bonds per square centimetre, preferably from 125 to 135 dot-bonds per square centimetre.

10. The laminate material (25) as claimed in any one of claims 6 to 9, **characterized in that** said adhesive is a polyurethane hot-melt adhesive.

11. The laminate material (25) as claimed in any one of the preceding claims, **characterized in that** the material exhibits a water vapour permeability value of at least 2500 g/m²/24h, preferably at least 3000 g/m²/24h more preferably at least 3500 g/m² /24h and most preferably at least 4000 g/m²/24h.

12. The laminate material (25) as claimed in any one of the preceding claims, **characterized in that** said first layer (26) and said second layer (28) have an amount of shrinkage which is no more than the amount of shrinkage of said third layer (30).

13. A garment (10) comprising the laminate material (25) of any one of the preceding claims.

14. The garment (10) as claimed in claim 13, **characterized in that** said garment is a diaper cover.

15. The garment (10) as claimed in claim 14, **characterized in that** the garment comprises a longitudinal axis (L) dividing the garment into a first lateral half (14) and a second lateral half (16);
a transverse axis (T) perpendicular to said longitudinal axis, said transverse axis dividing the garment into a first end (18) and a second end (20);
a fastening system for fastening the garment around the waist of a wearer, the fastening system having a first fastening arrangement (38) located on said first end (18) of the garment, said first fastening arrangement being located at an outer margin (40) of at least one of said first and said second lateral halves (14; 16) and comprising two outwardly facing edges (42; 44) forming an angle (α) therebetween, with said angle (α) being from 45 ° to 85 °, preferably from 50 ° to 80 °, more preferably from 55 ° to 75 ° and most preferably from 60 ° to 70 °.

16. The garment (10) as claimed in claim 14, **characterized in that** said garment has:
a peripheral edge (12)
a longitudinal axis (L) dividing the garment into a first lateral half (14) and a second lateral half (16);
a transverse axis (T) perpendicular to said longitudinal axis, said transverse axis dividing the garment into a first end (18) and a second end (20);
a fastening system for fastening the garment around the waist of a wearer, the fastening system having a first fastening arrangement (38) located on said first end (18) of the garment, said first fastening arrangement being located at an outer margin (40) of at least one of said first and said second lateral halves and comprising an outwardly facing edge (50), wherein said outwardly facing edge (50) is curved such that there exists two points on said outwardly facing edge, tangents to which subtend an angle (γ) of not more than 90 °, preferably not more than 60 ° and most preferably not more than 30 °.

17. The garment (10) as claimed in any one of claims 13 to 16, **characterized in that** said fastening system is a hook-and-loop fastening system.

18. The garment (10) as claimed in any one of claims 13 to 17, **characterized in that** said garment shrinks by no more than 6% after having been subjected to one hundred wash cycles at 60 °C.
